(19) Europäisches Patentamt
European Patent Office
Office européen des brevets

(11) Numéro de publication: **0 323 994 B1**

(12) **FASCICULE DE BREVET EUROPEEN**

(45) Date de publication de fascicule du brevet: **31.03.93**

(51) Int. Cl.5: **C07D 493/04**, C07D 307/20, C07H 19/01

(21) Numéro de dépôt: **88905905.1**

(22) Date de dépôt: **07.07.88**

(86) Numéro de dépôt internationale :
**PCT/FR88/00362**

(87) Numéro de publication internationale :
**WO 89/00162 (12.01.89 89/02)**

# (54) PROCEDE DE PREPARATION D'ANHYDRIDES D'HEXITOLS, D'HEXONOLACTONES, D'HEXOSES ET D'HEXOSIDES.

(30) Priorité: **07.07.87 FR 8709607**

(43) Date de publication de la demande:
**19.07.89 Bulletin 89/29**

(45) Mention de la délivrance du brevet:
**31.03.93 Bulletin 93/13**

(84) Etats contractants désignés:
**AT BE CH DE FR GB IT LI LU NL SE**

(56) Documents cités:
**EP-A- 0 052 295**
**EP-A- 0 061 055**

**Carbohydrate Research, Vol.136, 1985, Elsevier Science Publishers BV(Amsterdam ,NL), J.Defaye et al. :pages 53-65**

**Carbohydrate Research, Vol 152, 1986, Elsevier Science Publishers BV,(Amsterdam,NL) J.Defaye et al.: pages 89-98**

(73) Titulaire: **ERIDANIA BEGHIN-SAY**

**F-59239 Thumeries(FR)**

(72) Inventeur: **DEFAYE, Jacques**
**202, rue du Vercors**
**F-38330 Saint-Ismier(FR)**
Inventeur: **PEDERSEN, Christian**
**Bredesvinget 18**
**DK-2830 Virum(DK)**

(74) Mandataire: **David, Daniel**
**KAYSERBERG Service Propriété Industrielle**
**23 boulevard Georges Clemenceau**
**F-92402 Courbevoie Cédex (FR)**

EP 0 323 994 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Chemical Abstracts, Vol.106, N0.20, 18 May 1987, (Columbus,Ohio,US),page 14 & JP-A-61215621 (Sanyo Chemical Industries Ltd) 25 September 1986

Journal of Applied Polymer Science, Vol 33, No 4, March 1987, John Wiley & Sons, (New York, US) R.Franz et al, Pages 1291-1306

## Description

L'invention a pour objet un procédé amélioré pour la préparation de 1,4-3,6-dianhydro-hexitols per déshydratation acido-catalysée d'hexitols. Ce procédé concerne également la préparation de 3,6-anhydro-hexonolactones, de monoanhydro-hexitols, de 3,6-anhydro-hexoses ansi que d'alkyl-3,6-anhydro-hexosides.

Les dianhydrides d'hexitols sont connus depuis plus d'un siècle. Leurs méthodes de préparation ainsi que leurs propriétés ont été notamment rapportées dans plusieurs articles de revue (L.F. WIGGINS, advan. Carbohydr. Chem., 5 (1950) 191-228 ; S. SOLTZBERG, Advan. Carbohydr. Chem. Biochem. 25 (1970) 229-283 ; F. JACQUET, A. GASET et J.P. GORRICHON, Inform. Chim. 246 (1984) 155-158 ; G. FLECHE et M. HUCHETTE, Starch/Stärke, 38 (1986) 26-30). Les 1,4-3, 6-dianhydro-D-glucitol (isosorbide) {1} et les 1,4-3, 6-dianhydro-D-mannitol (isomannide) {2} sont les dianhydrides d'hexitols auxquels on se réfère le plus communément dans la littérature, puisque ce sont eux qui sont généralement obtenus partant de polyols commodément accessibles, tels le D-glucitol (sorbitol) et le D-mannitol.

Les dianhydrides d'hexitols sont usuellement préparés par action de solutions aqueuses d'acides minéraux à température élevée. Les rendements de ces réactions sont généralement médiocres, et il y a formation de produits indésirables. Des méthodes récentes ont proposé l'utilisation d'échangeurs de cations comme catalyseurs de déshydratation, en présence d'un solvant organique (J.C. GOODWIN, J.E. HODGE et D. WEISLEDER, Carbohyd. Res., 79 (1980) 133-141) ou encore sans solvant (J. FELDMANN, H. KOEBERNICK et H.U. WOELK, demande de brevet DE-A-3041673). Des halogénures d'hydrogène gazeux, en présence d'acides carboxyliques ou des anhydrides ou halogénures d'acides correspondants, ont également été proposés comme agents de déshydratation. Ainsi, la demande de brevet allemand DE-A-3111092 utilise le chlorure d'hydrogène gazeux à 140°. L'acide bromhydrique gazeux a été rapporté dans le même brevet comme donnant des résultats favorables ; enfin, on a mentionné l'utilisation possible de fluorure d'hydrogène gazeux. Par ailleurs, dans DE-A-3229412, on propose d'utiliser des acides protoniques ou de Lewis en réaction avec des diacyl-hexitols à des températures de 180-190°C.

Si les procédés d'obtention des dianhydro-polyols rapportés ainsi récemment conduisent bien à des rendements améliorés par rapport aux descriptions antérieures, il n'en reste pas moins que ces méthodologies utilisent des températures et des pressions souvent importantes qui augmentent les coûts de production et peuvent par ailleurs amener une corrosion du réacteur. Les résines anioniques utilisées dans un procédé alternatif sont des réactifs généralement coûteux, et pas toujours aisément recyclables, dans les réactions dans lesquelles elles sont mises en jeu.

L'invention se propose de remédier à ces inconvénients. Elle est caractérisée en ce que l'on fait réagir l'hexitol de départ avec au moins un acide carboxylique, ou un anhydride ou un halogénure d'acide correspondant, dans du fluorure d'hydrogène à l'état liquide.

On a constaté que la réaction de l'invention pouvait être étendue à l'obtention de monoanhydrides d'hexitols ainsi qu'à l'obtention d'anhydrides de composés apparentés aux hexitols. Ainsi, la réaction permet l'obtention de 3,6-anhydro-hexoses à partir d'hexoses ou de polysaccharides contenant ce motif. Le procédé de l'invention permet également d'obtenir des alkyl-3,6-anhydro-hexofuranosides.

Le procédé décrit dans la présente invention utilise le fluorure d'hydrogène à l'état liquide, en présence d'au moins un acide carboxylique ou d'un anhydride ou un halogènure d'acide correspondant.

La présente invention fournit un procédé de préparation de monoanhydrohexitols, de 1,4-3,6-dianhydro-hexitols, de 3,6-anhydro-hexonolactones et de 3,6-anhydro-hexoses à partir d'un produit de départ qui est, respectivement un hexitol, une hexonolactone, un hexose ou bien un polysaccharide contenant de telles unités monosaccharidiques, caractérisé en ce que l'on fait réagir ledit produit de départ dans du fluorure d'hydrogène liquide, avec au moins un acide carboxylique ou un anhydride ou un halogénure d'acide correspondant.

Pour des raisons de simplification de la terminologie, on sera amené à utiliser dans la présente description, le terme "acide carboxylique" pour désigner aussi bien l'acide carboxylique, que l'anhydride ou l'halogénure d'acide correspondant. De la même façon, l'expression "acide carboxylique" pourra représenter, le cas échéant, plusieurs acides carboxyliques.

L'acide formique est l'acide carboxylique le plus couramment utilisé en raison de son faible coût ainsi que de sa facilité d'élimination en fin de réaction. Des homologues supérieurs ou encore des acides carboxyliques de formule générale R-COOH peuvent néanmoins être utilisés, de même que les anhydrides d'acides carboxyliques ou les halogènures d'acides correspondants.

L'acide acétique, ainsi que ses homologues supérieurs, présentent toutefois l'avantage de pouvoir mieux contrôler la réaction, ce qui permet, en arrêtant celle-ci a un stade intermédiaire, d'améliorer l'accès aux monoanhydrides de polyols (1,4-anhydro-mannitol et 3,6-anhydro-sorbitol, respectivement).

L'action de l'acide carboxylique étant catalytique, it n'est pas nécessaire d'en utiliser une quantité équimoléculaire par rapport au composé de départ. Ainsi, le rapport molaire de l'acide carboxylique au composé de départ pourra être compris entre 0,1 et 1, sans qu'il soit exclu qu'un rapport plus faible ou plus élevé donne aussi des résultats. On a remarqué cependant que, lorsque la proportion d'acide carboxylique dépassait le seuil de l'équimolarité, ceci avait pour effet, en diluant le fluorure d'hydrogène, de diminuer corrélativement la vitesse de déshydratation du produit de départ mis en réaction et d'augmenter le temps d'obtention des anhydrides de ce produit. En effet, la concentration optimale du polyol par rapport au fluorure d'hydrogène se situe dans un rapport 1/4. Toutefois, il doit être entendu que, dans la pratique, la concentration peut varier autour de cette valeur optimale.

La réaction est généralement réalisée à température ambiante, température à laquelle le fluorure d'hydrogène qui a dissous les produits de départ est liquide. Cependant, une légère élévation de la température, à 40 ou 50 ° C, peut permettre de réduire le temps de réaction, dans le cas de la préparation de l'isosorbide en particulier.

Dans ce cas, un récipient clos doit être utilisé de façon à ce que le fluorure d'hydrogène reste sous forme liquide. De même, une température inférieure à la température ambiante peut être choisie, par exemple 0 ° C.

En fin de réaction, les réactifs sont éliminés par entraînement par un courant d'air ou d'un gaz inerte, suivi éventuellement d'un entraînement à la vapeur d'eau. Les résidus acides peuvent, lorsque cela est nécessaire, être neutralisés par action d'agents alcalins ou passage de la solution aqueuse sur résine échangeuse d'ions. Les anhydro-polyols peuvent être purifiés de façon classique par distillation et/ou cristallisation.

Les avantages apportés par la méthode de l'invention sont :

- Dépense énergétique moindre, la réaction pouvant se faire à température ambiante pour des temps de 20 minutes à 6 heures.
- Corrosion des réacteurs réduite par rapport aux acides minéraux usuels.
- Facilité d'isolement des produits ; HF est commode à éliminer. Il en est de même pour HCOOH si c'est l'acide carboxylique utilisé.
- Rendements et pureté des produits améliorés.
- Possibilité d'accès au 3,6- anhydro-D-glucitol (3,6-anhydro-sorbitol), première étape de la réaction avec le sorbitol, alors que les techniques classiques conduisent au 1,4-anhydride en première étape.
- Possibilité d'obtention du 1,4-anhydro-mannitol.

Une mise en oeuvre préférée du procédé de l'invention est décrite ci-dessous en ce qui concerne la déshydratation des polyols. Ce mode opératoire peut être appliqué de la même façon pour les produits apparentés utilisés comme produits de départ dans la présente invention. Une partie du polyol est additionnée d'environ quatre parties de fluorure d'hydrogène et d'une proportion équimoléculaire par rapport au polyol, d'un acide carboxylique, son anhydride ou l'halogénure d'acide correspondant. Le mélange réactionnel est alors conservé à température ambiante pendant des durées qui s'échelonnent de 20 minutes pour l'obtention d'isomannide {2} partant de D-mannitol, à 6 h pour l'obtention d'isosorbide {1} partant du sorbitol. On doit noter que, dans ce dernier cas, seul le 3,6-anhydro-sorbitol est formé au bout de 20 minutes et ce résultat diffère donc des méthodologies classiques utilisant les acides minéraux aqueux car, dans ces réactions, c'est le 1,4-anhydro-sorbitol qui est le premier produit formé, comme cela a été montré dans des articles publiés dans Acta Chem. Scand. B, 35 (1981) 441-449 et Starch/Stärke 38 (1986) 26-30. De plus, lorsque l'acide sulfurique ou des résines acides sont utilisés pour la déshydratation du mannitol, un mélange d'isomannide et de 2,5-anhydro-sorbitol, dans lequel prédomine ce dernier composé, est usuellement obtenu, alors qu'avec la méthodologie conforme à l'invention l'isomannide {2} est pratiquement le seul produit formé.

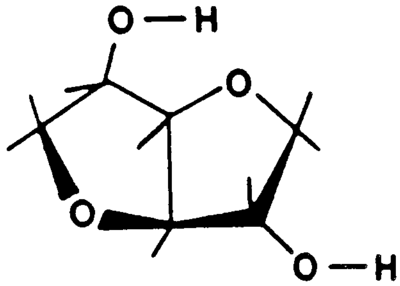

{1}

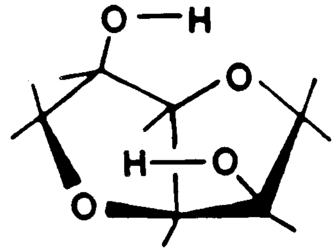

{2}

Les dianhydro-1,4-3,6-polyols sont obtenus avec des rendements de l'ordre de 60 % en produits cristallisés partant du sorbitol aussi bien que du mannitol, après purification des produits de la réaction par distillation et cristallisation. Dans la majorité des applications de ces produits, cependant, il est possible d'utiliser le produit brut de la réaction que contient à peu près uniquement les anhydro-polyols, après neutralisation éventuelle de l'acidité résiduelle.

Ce procédé de déshydratation intra-moléculaire de polyols a été étendu aux hexonolactones et en particulier aux D-gluconolactone {3} et D-mannonolactone {4}. Lorsque la D-mannonolactone est dissoute dans le fluorure d'hydrogène en présence d'une proportion équimoléculaire d'un acide carboxylique, la 3,6-anhydro-D-mannonolactone est obtenue au bout de 30 mn en rendement pratiquement quantitatif.

Un temps de deux heures à la même température est nécessaire pour transformer dans les mêmes conditions la D-gluconolactone en 3,6-anhydro-D-gluconolactone avec un rendement similaire.

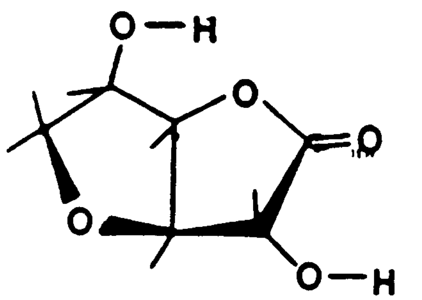

{3}

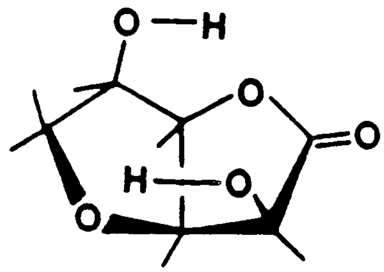

{4}

La présente invention permet également d'obtenir des alkyl-3,6-anhydro-hexofuranosides par un procédé dans lequel on fait réagir un hexose ou un polysaccharide contenant des unités monosaccharidiques dans du fluorure d'hydrogène liquide, avec au moins un acide carboxylique ou un anhydride ou un halogénure d'acide correspondant à une température inférieure à environ 50 ° C, caractérisé par le fait que l'on met en outre en réaction un alcool.

A titre indicatif, on peut citer les alcools aliphatiques ou cycloaliphatiques suivants :

- Les alcools primaires de formule R-OH où R est un groupement alkyle linéaire ou ramifié ayant de 1 à 20 atomes de carbone et comportant au moins un groupement cycloalkyle.
- Les alcools secondaires et tertiaires de formule R′OH où R′ est un groupement alkyle linéaire ou ramifié ayant de 3 à 20 atomes de carbone, ou un groupement cycloalkyle ou polycycloalkyle ayant de 3 à 20 atomes de carbone.
- Les diols et triols tels l'éthylène-glycol et le glycérol.

La demanderesse a constaté qu'il était préférable d'utiliser des alcools primaires, de préférence des alcools aliphatiques comportant de 1 à 4 atomes de carbone.

Il est nécessaire d'utiliser un rapport molaire alcool sur unités monosaccharidiques mises en jeu au moins égal à 1, un excès d'alcool permettant d'optimiser les rendements (l'alcool permettant en particulier d'entraîner les acidités résiduelles lors de l'évaporation et d'éviter la formation éventuelle de produits de reversion). Un rapport molaire alcool sur unités monosaccharidiques compris entre 2 et 20 sera préféré

(ceci permettant en particulier de minimiser la formation de produit de réversion oligosaccharidique).

On peut ainsi préparer des alkyl-3,6- anhydro-D-glucosides partant de D-glucose ou de ses précurseurs polysaccharidiques tels l'amidon ou la cellulose. Ce procédé s'applique à tout polysaccharide constitué d'unités hexoses qui seront susceptibles de conduire à la formation de 3,6-anhydro-hexosides. A titre d'exemple du procédé de l'invention, le D-glucose, l'amidon ou la cellulose sont additionnés d'environ quatre parties de fluorure d'hydrogène et d'une proportion équimoléculaire, par rapport aux unités glucopyranoses constitutives du polysaccharide, d'acide formique. Après deux heures à température ambiante, deux parties d'un alcool, tel que le méthanol sont ajoutées, de façon à minimiser la formation de produit de réversion et obtenir corrélativement les glycosides d'alkyle correspondants, selon les enseignements divulgués dans la demande de brevet WO 86/00906. Le fluorure d'hydrogène et le méthanol sont alors évaporés sous pression réduite et l'acidité résiduelle éventuelle est éliminée par entraînement par le méthanol et éventuellement neutralisation par addition de carbonate de calcium. Le méthyl-3,6- anhydro-D-glucofuranoside (produit de formule {5} où Alk = Me) est obtenu après distillation sous pression réduite avec un rendement de 50 à 60 %. Ce composé peut être transformé de façon classique, après hydrolyse acide douce suivie d'hydrogénation, en 3,6- anhydro-D-glucitol, ou par action du brome en présence de carbonate de calcium, en 3,6-anhydro-D-gluconolactone.

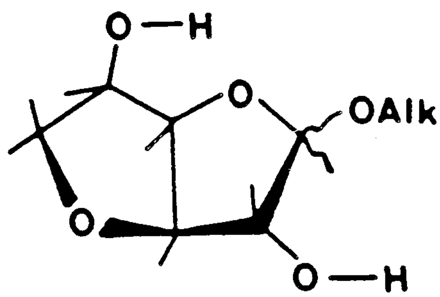

{5}

La présente invention permet aussi d'obtenir les dérivés mono- et diacylés des mono-anhydro-hexitols, de 1,4-3,6-dianhydro-hexitols, de 3,6-anhydro-hexofuranosides, c'est-à-dire les mono- et diesters de ces anhydrides.

De tels composés peuvent être obtenus en une étape par la présente invention.

En effet, en choisissant une stoéchiométrie d'au moins deux moles par rapport au polyol, à l'hexonolactone ou à l'hexose, de l'acide carboxylique, de l'anhydride d'acide ou de l'halogénure d'acide correspondant, on obtient préférentiellement le mono- ou le diester respectivement des anhydro-polyols, des 3,6-anhydro-hexonolactones et des alkyl-3,6-anhydro-hexosides.

Selon une variante de ce procédé, on peut ajouter une partie de l'acide carboxylique, de son anhydride ou de son halogénure après la séquence conduisant aux anhydrides objets de l'invention.

Selon une autre variante encore, l'acide carboxylique, l'anhydride ou l'halogénure utilisé pour l'estérification peut être différent de celui utilisé pour la préparation des anhydrides objets de l'invention.

Les dianhydrides d'hexitols sont des composés d'intérêt pour la préparation d'agents tensioactifs, émulsifiants, plastifiants ; comme inducteurs pour la préparation de polyéthers ou comme éléments structuraux pour les polyesters ; pour la préparation de polycarbonates, ainsi que comme agents durcissants pour la préparation de résines époxy. Ils sont également d'intérêt comme molécules pharmacophores. On peut prévoir des applications similaires pour les alkyl-3,6-anhydro-glycofuranosides et en particulier le méthyl-3,6- anhydro-D-glucofuranoside, qui possède des éléments de structure voisins, à savoir un système bicyclique tétrahydrofurannique condensé, portant deux fonctions alcool.

Dans une partie des applications présentées ci-dessus, on utilise dans les compositions les mono- ou diesters de ces anhydrides.

Les exemples suivants, donnant des modes opératoires type pour préparer certains des produits revendiqués, illustrent l'invention sans la limiter.

Exemple 1

**Préparation du 1,4-3,6- dianhydro-D-**mannitol (Isomannide).

Dans un récipient en polyéthylène ou en acier, on place le mannitol (5 g), l'acide formique 1,1 ml (1 équivalent molaire) et le fluorure d'hydrogène (20 ml). Au bout de 20 mn à 20°, un spectre de $^{13}$C-r.m.n. indique que le mannitol est transformé quantitativement en le dérivé formylé du 1,4-3,6- d ianhydro-D-mannitol. Le fluorure d'hydrogène et l'acide formique sont alors évaporés, soit par passage d'un courant d'air, soit sous pression réduite. Une substance huileuse est obtenue (3,7 g, 92 % calc. en anhydromannitol), qui contient au moins 80 % d'isomannide. Une distillation de cette huile sous une pression de 1 mm conduit à 2,2 g (55 %) de 1,4-3,6- dianhydro-D-mannitol, p.f. 84-86°, eb. 110° sous 1 mm Hg.

Note : L'élimination complète du fluorure d'hydrogène peut être réalisée avant distillation de l'isomannide, par coévaporation avec l'eau du résidu, puis passage de la solution aqueuse sur colonne de résine "Amberlite IR 45 ($OH^-$)".

Exemple 2

**Préparation du 1,4-3,6-dianhydro-sorbitol (Isosorbide).**

Le mode opératoire précédent est utilisé en partant de sorbitol (5g). Au bout de 20 mn de réaction, on remarque que le sorbitol a disparu du milieu réactionnel, mais que pratiquement seul le 3,6-anhydro-sorbitol est formé. Ce composé peut être isolé à ce stade par évaporation du fluorure d'hydrogène. L'obtention de 1,4-3,6-dianhydro-sorbitol (isosorbide) demande de prolonger la réaction pendant 6 h à température ambiante. Ce temps de réaction peut être réduit à 3 h par chauffage à 40°C. Le rendement de la réaction est identique à celui de l'isomannide.

L'isosorbide obtenu après distillation (2,3 g, 57 %) a un point de fusion de 61-63°.

Exemple 3

**Préparation du 3,6- anhydro-D-sorbitol**

Le sorbitol (5 g) est additionné d'anhydride acétique (3,1 ml, 2 équivalents moléculaires) et de fluorure d'hydrogène (20 ml). La solution est maintenue à 20°C pendant 48 h puis HF est évaporé par passage d'un courant d'air ou sous pression réduite, et le résidu est co-évaporé à trois reprises par l'eau et ensuite par le méthanol. Le résidu ainsi obtenu est alors traîté par une quantité catalytique de méthylate de sodium dans le méthanol (~ 100 mg dans 50 ml de méthanol) et, après 30 mn, la solution est deminéralisée par agitation avec une résine acide (Amberlite IR120 H+). L'évaporation de la solution méthanolique conduit à 3,2 g d'un résidu qui contient le 3,6-anhydro-sorbitol (88 %) et l'isosorbide (12 %, $^{13}$C r.m.n.). Une cristallisation dans un mélange éthanol-acétate d'éthyle permet d'isoler le 3,6-anhydro-sorbitol (2.1 g, 47 % ; p.f. 100°C). Par recristallisation, ce composé fond à 110-112° ; $[\alpha]_D^{20}$ -7,3 (c 1,7, eau).

Exemple 4

**Préparation du 1,4- anhydro-D-mannitol**

Le mode opératoire précédent est utilisé partant du mannitol (5 g). Le résidu (4,5 g) obtenu après évaporation de HF, co-évaporations et action de méthylate de sodium contient le 1,4- anhydro-D-mannitol (76 %) et l'isomannide (22 %); une cristallisation dans un mélange éthanol-acétate d'éthyle permet d'isoler le 1,4-anhydride (2,86 g, p.f. 134-137°C). Une recristallisation porte ce point de fusion à 143-145°C, $[\alpha]_D^{20}$ -23,7 (c 2,5,eau).

Exemple 5

**Préparation de la 3,6- anhydro-D-glucono-1,4-lactone**

La D-glucono-1,5-lactone (5 g) est additionnée d'acide formique (0,5 ml) et de fluorure d'hydrogène (20 ml). Après 2 h à 20°C, un spectre de $^{13}$C-r.m.n. indique que la transformation de la lactone de départ est complète. HF et $HCO_2H$ sont alors évaporés par passage d'un courant d'air ou sous pression réduite.

L'anhydro-lactone est recristallisée dans l'acétate d'éthyle (2,7g, 62 %, p.f. 112-114°).

Exemple 6

**Préparation de la 3,6- anhydro-D-mannono-1,4-lactone**

Le mode opératoire précédent est appliqué à la D-mannono-1,4-lactone (2,5 g). Le temps de réaction est plus court (30 mn) et l'anhydro-lactone obtenue avec un rendement de 91 % (2,3 g, p.f. 108-110°C).

Exemple 7

**Préparation de méthyl-3,6-anhydro-α, β-D/B\-glucofuranosides et de 3,6-anhydro-D-glucose**

Le D-glucose ou l'amidon, à 10 % d'humidité, (5 g) sont additionnés de fluorure d'hydrogène (20 ml) et d'acide formique (0,5 ml). Après 2 h à température ambiante, du méthanol (10 ml) est ajouté avec refroidissement simultané, puis HF, $HCO_2H$ et le méthanol, sont éliminés par passage d'un courant d'air, ou sous pression réduite. L'acidité résiduelle est éliminée par entraînement par le méthanol sec puis neutralisation par le carbonate de calcium. Le mélange de méthyl-3,6-anhydro-α-et-β- D-glucofuranosides (4,4 g, 89 %) est obtenu sous forme d'une huile, après distillation du produit de la réaction sous un vide de 4 x $10^{-2}$ mmHg (ébullition 140-150°C). Le mélange s'hydrolyse spontanément à température ambiante dans l'acide sulfurique 0,1 N en 3,6-anhydro-D-glucose (p.f.121°C). Ce composé peut être transformé en 3,6-anhydro-D-glucitol par hydrogénation, selon les conditions classiques utilisées pour l'obtention du polyol, et en 3,6-anhydro-D-glucono-1,4-lactone par action du brome en solution aqueuse en présence de carbonate de calcium.

Exemple 8

**Préparation d'octyl-3,6-anhydro-α, β-D-glucofuranosides**

Le D-glucose ou l'amidon (10 % d'humidité) (5 g), sont additionnés d'acide formique (0,5 ml) et de fluorure d'hydrogène (20 ml). Après deux heures à température ambiante, l'octanol (8 ml) est ajouté. Le mélange est alors concentré sous pression réduite à 50°C. Le résidu sec est repris par l'eau et neutralisé par addition d'une solution aqueuse d'hydroxyde de sodium. La solution aqueuse est extraite par le dichlorométhane et la solution chlorométhylénique est séchée sur sulfate de sodium puis concentrée. Le mélange d'octyl-3,6-anhydro-α et β-D-glucofuranosides est obtenu sous la forme d'une huile (2,28 g, 30 %)-.R.m.n. $^{13}C$ : δ(p.p.m.) 110,6 (C-1,β), 103,9 (C-1, α), 87,4 (C-4,α),87,2 (C-4,β), 83,6, 79,9 (C-2, C-3, β), 78,7, 77,3 (C-2,C-3,α), 71,5 71,1 (C-5,C-6,β), 71,1, 70,4 (C-5 C-6,α), 68,2, 32,4, 31,8, 29,4 (2C), 25,8, 22,5, 13,6 (C-octyle).

## Revendications

1. Procédé de préparation de monoanhydro hexitols, de 1,4-3,6-dianhydro-hexitols, choisis parmi le 1,4:3,6-dianhydro-D-mannitol (isomannide), le 1,4:3,6-dianhydro-D-sorbitol (isosorbide), le 1,4-anhydro-D-mannitol et le 3,6-anhydro-D-sorbitol, de 3,6-anhydro-hexonolactones et de 3,6-anhydro-D-glucose à partir d'un produit de départ qui est, respectivement, un hexitol, une hexonolactone et le D-glucose, caractérisé en ce que l'on fait réagir ledit produit de départ dans du fluorure d'hydrogène liquide, avec au moins un acide carboxylique ou un anhydride ou un halogénure d'acide correspondant, à une température inférieure à environ 50°C.

2. Procédé selon la revendication 1, caractérisé en ce que ledit acide carboxylique est l'acide formique.

3. Procédé selon l'une des revendications 1 et 2, caractérisé en ce que le rapport molaire dudit acide carboxylique ou anhydride ou halogénure d'acide correspondant par rapport audit produit de départ est compris entre 0,1 et 2.

4. Procédé selon la revendication 3, caractérisé en ce que ledit rapport molaire dudit acide carboxylique ou anhydride ou halogénure d'acide correspondant audit produit de départ est compris entre 0,5 et 1.

5. Procédé de préparation de monoanhydro-hexitols selon la revendication 1, caractérisé en ce que ledit acide carboxylique est l'acide acétique.

6. Procédé de préparation d'alkyl-3,6-anhydro-hexofuranosides dans lequel on fait réagir un hexose ou un polysaccharide contenant de telles unités monosaccharidiques dans du fluorure d'hydrogène liquide, avec au moins un acide carboxylique ou un anhydride ou un halogénure d'acide correspondant, à une température inférieure à environ 50 ° C, caractérisé en ce que l'on met en outre en réaction un alcool.

7. Procédé selon la revendication 6, dans lequel ledit alcool est un alcool aliphatique primaire ayant 1 à 4 atomes de carbone.

8. Procédé permettant d'obtenir préférentiellement les dérivés mono- et diacylés des produits préparés selon la revendication 1 ou selon la revendication 6, caractérisé en ce que le rapport molaire dudit acide carboxylique ou anhydride ou halogénure d'acide correspondant par rapport audit produit de départ est au moins égal à 2.

9. Procédé de préparation selon la revendication 8 de dérivés mono- et diacylés de monoanhydrohexitols, de 1,4-3,6-dianhydro-hexitols, de 3,6-anhydro-hexonolactones et de 3,6-anhydrohexoses selon la revendication 1 dans lequel on fait réagir un produit de départ qui est, respectivement, un hexitol, une hexonolactone et un hexose, ou bien un polysaccharide contenant de telles unités monosaccharidiques dans du fluorure d'hydrogène liquide, avec au moins un acide carboxylique ou un anhydride ou un halogénure d'acide correspondant, dans un rapport molaire par rapport audit produit de départ au moins égal à 2, caractérisé par le fait que l'on ajoute une partie dudit acide carboxylique, anhydride ou halogénure d'acide après la séquence conduisant à l'obtention d'anhydrides initialement formés.

10. Procédé de préparation selon la revendication 8 de dérivés mono- et diacylés d'alkyl-3,6-anhydro-hexofuranosides selon la revendication 6 dans lequel on fait réagir un hexose ou bien un polysaccharide contenant de telles unités monosaccharidiques dans du fluorure d'hydrogène, avec au moins un acide carboxylique ou un anhydride ou un halogénure d'acide correspondant, dans un rapport molaire par rapport audit produit de départ au moins égal à 2 et dans lequel on met en outre en réaction un alcool, caractérisé par le fait que l'on ajoute une partie dudit acide carboxylique anhydride ou halogénure d'acide après la séquence conduisant à l'obtention de l'anhydride initialement formé.

11. Procédé selon l'une des revendications 8 et 9, caractérisé en ce que ledit acide carboxylique, anhydride d'acide ou halogénure d'acide est différent de celui utilisé pour l'estérification desdits anhydrides.

12. Octyl-3,6-anhydro-$\alpha,\beta$-D-glucofuranosides obtenu selon le procédé de la revendication 6.

## Claims

1. A method for the preparation of monoanhydrohexitols, 1,4-3,6-dianhydrohexitols, selected from 1,4:3,6-dianhydro-D-mannitol (isomannide), 1,4:3,6-dianhydro-D-sorbitol (isosorbide), 1,4-anhydro-D-mannitol and 3,6-anhydro-D-sorbitol, 3,6-anhydrohexonolactones and 3,6-anhydro-D-glucose, from a starting material which is a hexitol, a hexonolactone and D-glucose respectively, characterised in that the said starting material is reacted in liquid hydrogen fluoride with at least one carboxylic acid or a corresponding anhydride or halide of the acid at a temperature below about 50 ° C.

2. A method according to claim 1, characterised in that the said carboxylic acid is formic acid.

3. A method according to either of claims 1 or 2, characterised in that the molar ratio of the said carboxylic acid or corresponding anhydride or halide of the acid in relation to the starting material lies between 0.1 and 2.

4. A method according to claim 3, characterised in that the said molar ratio of the said carboxylic acid and corresponding anhydride or halide of the acid to the said starting material lies between 0.5 and 1.

**5.** A method of preparing monoanhydrohexitols according to claim 1, characterised in that the said carboxylic acid is acetic acid.

**6.** A method for preparing alkyl-3,6-anhydrohexofuranosides in which a hexose or polysaccharide containing such monosaccharide units is reacted in liquid hydrogen fluoride with at least one carboxylic acid or a corresponding anhydride or halide of the acid at a temperature below about 50 °C, characterised in that an alcohol is also involved in the reaction.

**7.** A method according to claim 6, in which the said alcohol is a primary aliphatic alcohol having from 1 to 4 carbon atoms.

**8.** A method by means of which the mono- and diacyl derivatives of materials prepared according to claim 1 or according to claim 6 may preferentially be obtained, characterised in that the molar ratio of the said carboxylic acid or corresponding anhydride or halide to the said starting material is equal to at least 2.

**9.** A method for the preparation according to claim 8 of mono- and diacyl monoanhydrohexitol derivatives, 1,4-3,6-dianhydrohexitols, 3,6-anhydro-hexenolactones and 3,6-anhydrohexoses according to claim 1 in which a starting material which is a hexitol, a hexonolactone and a hexose respectively or a polysaccharide containing such monosaccharide units is reacted in liquid hydrogen fluoride with at least one carboxylic acid or corresponding anhydride or halide of the acid in a molar ratio with respect to the said starting material of at least 2, characterised in that part of the said carboxylic acid, anhydride or halide of the acid is added after the sequence leading to the production of the anhydrides initially formed.

**10.** A method of preparation according to claim 8 of mono- and diacyl derivatives of alkyl-3,6-anhydrohexofuranosides according to claim 6, in which a hexose or a polysaccharide containing such monosaccharide units is reacted in hydrogen fluoride with at least one carboxylic acid or a corresponding anhydride or halide of the acid in a molar ratio with respect to the said starting material of at least 2 and in which an alcohol is also involved in the reaction, characterised in that one part of the said carboxylic acid or anhydride or halide of the acid is added after the sequence leading to the formation of the anhydride initially formed.

**11.** A method according to one of claims 8 and 9, characterised in that the said carboxylic acid, anhydride or halide of the acid is different from that used for esterification of the said anhydrides.

**12.** Octyl-3,6-anhydro-α, β-D-glucofuranosides obtained according to the method in claim 6.

**Patentansprüche**

**1.** Verfahren zur Herstellung von Monoanhydro-Hexitolen, 1,4-3,6-Dianhydro-Hexitolen, die unter 1,4:3,6-Dianhydro-D-Mannitol (Isomannid), 1,4:3,6-Dianhydro-D-Sorbitol (Isosorbid), 1,4-Anhydro-D-Mannitol und 3,6-Anhydro-D-Sorbitol ausgewählt sind, von 3,6-Anhydro-Hexonolactonen und von 3,6-Anhydro-D-Glukose auf der Basis eines Ausgangsstoffes, der jeweils ein Hexitol, ein Hexonollacton und D-Glukose ist, dadurch gekennzeichnet, daß man den Ausgangsstoff in flüssigem Fluorwasserstoff mit mindestens einer Karbonsäure oder einem Anhydrid oder einem entsprechenden Säurehalogenid bei einer Temperatur unter etwa 50 ° C reagieren läßt.

**2.** Verfahren nach Anspruch 1, dadurch gekennzeichnet, daß die Karbonsäure Ameisensäure ist.

**3.** Verfahren nach einem der Ansprüche 1 und 2, dadurch gekennzeichnet, daß der molare Anteil der Karbonsäure oder des Anhydrides oder des entsprechenden Säurehalogenides in bezug auf den Ausgangsstoff zwischen 0,1 und 2 beträgt.

**4.** Verfahren nach Anspruch 3, dadurch gekennzeichnet, daß der molare Anteil der Karbonsäure oder des Anhydrides oder des entsprechenden Säurehalogenides in bezug auf den Ausgangsstoff zwischen 0,5 und 1 beträgt.

5. Verfahren zur Herstellung von Monoanhydro-Hexitolen nach Anspruch 1, dadurch gekennzeichnet, daß die Karbonsäure Essigsäure ist.

6. Verfahren zur Herstellung von Alkyl-3,6-Anhydro-Hexofuranosiden, bei dem man eine Hexose oder ein Polysaccharid, das solche monosacharidischen Einheiten enthält, in Fluorwasserstoff mit mindestens einer Karbonsäure oder einem Anhydrid oder einem entsprechenden Säurehalogenid bei einer Temperatur unter etwa 50° C reagieren läßt, dadurch gekennzeichnet, daß man des weiteren einen Alkohol in die Reaktion einführt.

7. Verfahren nach Anspruch 6, bei dem der Alkohol ein primärer aliphatischer Alkohol mit 1 bis 4 C-Atomen ist.

8. Verfahren, mit dem man mono- und diacylierte Derivate von gemäß Anspruch 1 oder gemäß Anspruch 6 hergestellten Produkten herstellen kann, dadurch gekennzeichnet, daß der molare Anteil der Karbonsäure oder des Anhydrides oder des entsprechenden Säurehalogenides in bezug auf den Ausgangsstoff mindestens 2 beträgt.

9. Verfahren nach Anspruch 8 zur Herstellung von mono- und diacylierten Derivaten von Monoanhydrohexitolen, 1,4-3,6-Dianhydro-Hexitolen, 3,6-Anhydro-Hexanolactonen und 3,6-Anhydrohexosen gemäß Anspruch 1, bei dem man einen Ausgangsstoff, der jeweils ein Hexitol, ein Hexonolacton und eine Hexose oder ein solche monosaccharidischen Einheiten enthaltendes Polysaccharid ist, in flüssigem Fluorwasserstoff mit mindestens einer Karbonsäure oder einem Anhydrid oder einem entsprechenden Säurehalogenid mit einem molaren Anteil in bezug auf den Ausgangsstoff reagieren läßt, der mindestens 2 beträgt, dadurch gekennzeichnet, daß man nach der Verfahrenssequenz, die zur Herstellung der anfangs gebildeten Anhydride führt, einen Teil der Karbonsäure, des Anhydrides oder des Säurehalogenides zusetzt.

10. Verfahren nach Anspruch 8 zur Herstellung von mono- und diacylierten Derivaten von Alkyl-3,6-Anhydro-Hexofuranosiden gemäß Anspruch 6, bei dem man eine Hexose oder ein solche monosaccharidischen Einheiten enthaltendes Polysaccharid in Fluorwasserstoff mit mindestens einer Karbonsäure oder einem Anhydrid oder einem entsprechenden Säurehalogenid in einem molaren Anteil in bezug auf den Ausgangsstoff von mindestens 2 reagieren läßt und des weiteren in die Reaktion einen Alkohol einführt, dadurch gekennzeichnet, daß man nach der zur Herstellung des anfangs gebildeten Anhydrides durchgeführten Verfahrenssequenz einen Teil der Karbonsäure, des Anhydrides oder Säurehalogenides zusetzt.

11. Verfahren nach einem der Ansprüche 8 und 9, dadurch gekennzeichnet, daß sich die Karbonsäure, das Säureanhydrid oder Säurehalogenid von dem unterscheidet, das für die Veresterung der Anhydride verwendet wurde.

12. Octyl-3,6-Anhydro-α, β-D-Glukofuranoside erhalten nach dem Verfahren gemäß Anspruch 6.